# EUROPÄISCHE PATENTSCHRIFT

(11) **EP 0 725 816 B1**
(45) Veröffentlichungstag und Bekanntmachung des Hinweises auf die Patenterteilung: **15.03.2000**
(21) Anmeldenummer: 94930220.2
(22) Anmeldetag: 25.10.1994
(51) Int. Cl.: C12N 5/06, C12N 5/08

(54) **VERFAHREN ZUM ZÜCHTEN VON BIPOLAR ADHÄRIERTEN HEPATOZYTEN**
METHOD FOR CULTURING BIPOLAR-ADHESION HEPATOCYTES
PROCEDE DE CULTURE D'HEPATOCYTES A ADHERENCE BIPOLAIRE

(30) Priorität: 26.10.1993 DE 4336399
(43) Veröffentlichungstag der Anmeldung: 14.08.1996
(73) Patentinhaber: BADER, Augustinus, D-31275 Lehrte (DE)
(72) Erfinder: BADER, Augustinus, D-31275 Lehrte (DE)
(74) Vertreter: Lorenz, Werner, Dipl.-Ing.
(86) Internationale Anmeldenummer: EP9403501
(87) Internationale Veröffentlichungsnummer: WO9511963

(56) Entgegenhaltungen:
- WO-A-93/18133
- WO-A-94/01535
- FASEB JOURNAL, Bd.3, Nr.2, Februar 1989, BETHESDA, MD US Seiten 174 - 177 J.C.Y. DUNN ET AL. 'HEPATOCYTE FUNCTION AND EXTRACELLULAR MATRIX GEOMETRY: LONG-TERM CULTURE IN A SANDWICH CONFIGURATION'
- EUROPEAN JOURNAL OF BIOCHEMISTRY, Bd.213, Nr.2, April 1993, BERLIN, DE Seiten 805 - 814 B. SAAD ET AL. 'CRUDE LIVER MEMBRANE FRACTIONS AND EXTRACELLULAR MATRIX COMPONENTS AS SUBSTRATA REGULATE DIFFERENTIALLY THE PRESERVATION AND INDUCIBILITY OF CYTOCHROME P-450 ISOENZYMES IN CULTURED RAT HEPATOCYTES.'
- CHEMICAL ABSTRACTS, vol. 108, no. 5, 1. Februar 1988, Columbus, Ohio, US; abstract no. 34404y, N. SAWADA ET AL. 'EFFECTS OF EXTRACELLULAR MATRIX COMPONENTS ON THE GROWTH AND DIFFERENTIATION OF CULTURED RAT HEPATOCYTES.' Seite 320 ; & IN VITRO CELL. DEV. BIOL., Bd.23, Nr.4, 1987 Seiten 267 - 273
- EXPERIMENTAL CELL RESEARCH, Bd.208, Nr.2, Oktober 1993, NEW YORK, N.Y., US Seiten 442 - 452 R.M. EZZELL ET AL. 'EFFECT OF COLLAGEN GEL CONFIGURATION ON THE CYTOSKELETON IN CULTURED RAT HEPATOCYTES.'
- JOURNAL OF HEPATOLOGY, Bd.16, Nr.SP.1, 1992, AMSTERDAM, NL Seite S78 A. BADER ET AL. 'THE SANDWICH CULTURE TECHNIQUE FOR PRIMARY HEPATOCYTES - TOMORROW'S GOLDEN STANDARD?'

## Beschreibung

Die Erfindung betrifft ein Verfahren zur Verbesserung der Matrixbedingungen bipolar adhärierter Hepatozyten und zur Herstellung eines entsprechend konfigurierten Zellkulturkits.

Die Verwendung von primär komplexen Matrixsubstanzen, wie sie von Sarcomen abgeleitet werden (Matrigel), ist für die Kultur von Hepatozyten nachteilig und für Untersuchungen die die in vivo Situation repräsentieren sollen, nicht sinnvoll, da sie zu qualitativen Veränderung dieser Zellen führen wie z.B. der Morphologie oder der Cytochromexpression.

Weiterhin können in einem derartigen System nur unzureichend molekularbiologische Untersuchungen durchgeführt werden, da durch die Gewinnung der Matrixkompenenten aus einem Sarkom ein nicht unerheblicher Anteil von kontaminierender DNA und RNA spaltenden Enzymen (RNAsen) notgedrungen in das Kulturmodell miteingebracht wird.

Wurden derartige primär komplexe Matrixen nicht verwendet, so wurden diese Matrixkomponenten bisher dem Kulturüberstand von nach der Einzelgeltechnik kultivierten Hepatozyten zugegeben, oder auch innerhalb der Matrix von Einzelgelsytemen verwendet. Trotz dieser Bemühungen ließen sich keine dem Potential der Hepatozyten entsprechenden Effekte erreichen, da die hierbei verwendete Hepatozytenkulturtechnik nicht den Anforderungen der Hepatozyten an ein in vitro System entsprach. Hepatozyten benötigen in vitro ein bipolare Adhäsion an extrazelluläre Matrix, wodurch die in vivo Situation innerhalb des Disseschen Spaltraums imitiert werden kann. Derartige Bestrebungen fanden Niederschlag in der sogenannten Sandwich-technik oder auch modifizierte Gel Entrapment Technik. Das Gemeinsame dieser beiden Techniken liegt darin, daß die Hepatozyten in einem Monolayer eingebettet werden, umgeben von zwei Schichten. Diese Einbettung wurde bisher mit einer einfachenn Grundmatrix, basierend auf Kollagen Typ I aus Rattenschwanzkollagen, hergestellt. Diese Techniken sind aber immer noch defizitär hinsichtlich des eigentlichen Ziels, der möglichst in vivo artigen Zusammensetzung der extrazellulären Matrix.

Ein Verfahren dieser Art ist z.B. in der DE 42 06 585 beschrieben.

Dies bedingt eine Reihe von wesentlichen Nachteilen wie z.B. die Unmöglichkeit diese Zellen ohne Zugabe zusätzlicher externer Faktoren mittels des Kutlurmediums innerhalb derartiger Substrate serumfrei kultivieren zu können. So müssen zusätzlich durch das Kulturmedium mittels FCS oder speziell adaptierten Kulturmedien fehlende Faktoren hinzugegeben werden. Weiterhin fehlen in diesen einfachen Grundmatrixen Adhäsionssubstrate wie sie in vivo jedoch ein bedeutende Rolle spielen. Funktionelle Veränderungen der Hepatozyen können dadurch induziert werden.

Die serumfreie Kultur von Hepatozyten wurde bisher durch Verwendung spezieller Kulturmedien erreicht, die als überstand zu bereits adhärenten oder sich im Adhäsionsprozess befindlichen Zellen hinzugegeben wurden. Diese speziell entwickelten Kulturmedien enthalten zum Teil sehr teure Zusätze deren Konzentrationen notgedrungen durch das Volumen des Kulturmediums verdünnt werden und die auch wegen des in regelmäßigen Abständen erforderlichen Wechsels von Kulturmedium immer wieder erneut hinzugegeben werden. So ist z.B. bei primären Hepatozyten der Medienwechsel alle 24 h bis 48 h erforderlich. Diese Kulturemedien werden aus historischen Gründen weiterhin in unnötiger Weise und Unkenntnis Faktoren zugestetzt wie sie für eine längerfristigen serumfreie Kultur nicht benötigt werden. Hierzu zählt z.B. Transferrin, Hormone, Hypohysenextrakte und andere.

Ein universelles Verfahren, bei dem relevante Matrixkomponenten zu den bipolar adhärierten Hepatozyten im unmittelbaren Adhäsionsbereich gebracht werden könnten, würde die Kultur erleichtern und vorteilhaftere Bedingungen in der Mikroumgebung der Zellen schaffen.

Ein Vorteil eines derartigen universellen Verfahrens wäre die Einsparung von Kosten und Ressourcen. Diese Sandwichtechniken alleine reichen zur serumfreien Kultur dieser Zellen jedoch nicht aus. Bisher war noch kein Verfahren bekannt, das in direkten Bezug für die Herstellung der Sandwichtechnik (Sandwichtechnik) oder Gel Entrapment Technik (Gel Entrapment Technik) die serumfreie Kultur von Hepatozyten ermöglicht, ohne hierbei auf die Kombination von speziell adaptierten Medien angewiesen zu sein. Diese sind im kommerziellen Sektor auch als zur serumfreien Kultur geeignete Medien bezeichnet. Weiterhin gibt es bisher keinen Zellkulturkit, der die Sandwichtechnik für primäre Hepatozyten an sich ermöglicht.

Als Grundmatrix der Sandwichtechnik oder Gel Entrapment Technik dient üblicherweise Kollagen Typ I, das z.B. aus Rattenschwänzen oder Rinderhaut in herkömmlicher Weise präpariert werden kann. Am Ende des Herstellungsprozesses wird der pH dieser Kollagenlösung auf ca. 4 eingestellt und in dieser Form bei 4 °C aufbewahrt. Auch die Herstellung eines Kollagen Lyophilisats in herkömmlicher Weise ist möglich. Bei der Sandwichtechnik bzw. Gel Entrapment Technik wird die saure Matrix mit einem basischen Medium-konzentrat (üblicherweise ein 10x Konzentrat) im Verhältnis 1:10 gemischt. Dieses Mediumkonzentrat dient als Katalysator zur Induktion der Gelierung der Matrix und wird im Gelierungsprozess in die Grundmatrix gebunden. Wird nach der Sandwichtechnik weiter vorgegangen, so wird diese Mixtur auf die zur Kultur vorgesehenen Flächen aufgegeben. Nach der Gelierung werden die Zellen aufgetragen und nach deren Adhäsion wird der Überstand, der auch die nicht adhärenten, toten Zellen enthält, entfernt und die zweite Matrixschicht daraufgegeben. Nach erfolgter Geherung dieser zweiten Schicht wird nun das Kulturmedium (normalerweise 2-4 ml, 28 cm²) auf die verfestigte, obere Matrixschicht aufgegeben. Dieses Kulturmedium wird in regelmäßigen Abständen gewechselt, um einerseits entsprechende Messungen im Überstand vornehmen zu können, und weil andererseits eine Anreicherung von Stoffwechselend-produkten im Überstand stattfindet. Bei der Gel Entrapment Technik Technik werden die Zellen sofort in die kalte, noch flüssige Matrix eingegeben und in diesem Zustand auf die zur Kultivierung vorgesehenen Oberflächen aufgegossen. Nach der Verfestigung dieser Zell-Matrix Schicht wird, wie auch bei der Sandwichtechnik, Kulturmedium oben aufgegeben. Das anfangs beschriebene Mediumkonzentrat ist in der festen (stationären) Phase primär gebunden und ist somit nicht zu verwechseln mit dein als flüssige Phase hinzugegebenen Kulturmedium. Dieses ist durch Abpipettieren einfach und schnell austauschbar.

Sowohl die Sandwichtechnik als auch die Gel Entrapment Technik (Immobilisationstechnik) stabilisieren die Funktion von Hepatozyten in Kultur. Eigene, unveröffentlichte Vorarbeiten haben gezeigt, daß selbst bei Verwendung von Standard Kulturmedien wie z.B. Williams E, die primär nicht für serumfreie Kulturen konzipiert wurden, die serumfreie Kultur auch für Hepatozyten in der Sandwichtechnik oder Gel Entrapment Technik möglich ist, wenn Serum zumindest während der Adhäsionsphase dieser Zellen an extrazelluläre Matrix für einige Stunden dem Kulturmedium (Überstand) beigesetzt wird. Dies stellt jedoch eine konventionelle Technik dar, FCS wird in herkömmlicher Weise dem Medium zugesetzt. Ohne die Verwendung von FCS und bei Standardmedien wie z.B. Williams E oder DMEM verlieren diese Zellen trotz allem rasche Ihre Funktion.

In Abhängigkeit von der Serum Charge, die üblicherweise von mehreren Tieren gepoolt wird, können jedoch negative Einflüsse hinsichtlich der Überlebenszeit und der Funktionsfähigkeit in Kultur auftreten. Dies ist abhängig von z.B. Endotoxingehalt und anderen nicht näher identifizierbaren Zusätzen im Serum, das üblicherweise von Kälbern gewonnen wird, die Verwendung von Serum einer anderen Spezies ist jedoch auch möglich. Die mangelnde Definierbarkeit bedingt eine mangelnde Reproduzierbarkeit von Versuchsergebnissen. So kann unter Verwendung von FCS zwar eine längerfristige Kultur nach der Sandwichtechnik erreicht werden, die Untersuchungen sind jedoch nach eigenen Daten insbesonders bei rezeptorvermittelten Studien stark chargenabhängig. Dies führte bei Untersuchungen zur Wachststumsstimulation der Sandwichtechnik unter Verwendung eines rekombinanten epidermalen Wachstumsfaktors (EGF) zu einem Mißerfolg der Studien von ca. 50-60 % innerhalb eines Zeitraums von 2 Jahren.

In bisherigen Publikationen über die Sandwichtechnik wird nicht auf derartige Probleme eingegangen und nur endogene Prozesse des Hepatozyten, wie z.B. die Albuminsekretion dargestellt. Bei Zusatz von FCS steigt die Albuminsekretion nach einer initial niedrigen Rate (bedingt durch den postisolatorischen Traumatisationszustand der durch die Zellisolation verursacht wird) zu dem für die Sandwich-technik stabilen Plateau an. Bei Weglassen des FCS und Verwendung einer Sandwichtechnik unterscheiden sich die Sekretionsraten nicht innerhalb der ersten Tage in Kultur. Dies bedeutet, daß für eine kurzfristige Kultur matrixüberschichteter Hepatozyten ein Zusatz von Fibronektin nicht nötig ist, und dadurch kein erkennbarer Vorteil hinsichtlich der Albuminsekretisonsrate zu erzielen ist.

Auch nicht überschichtete Hepatozyten (konventionelle Kulturtechnik) können serumfrei mit oder ohne FCS und ohne FCS aber mit Fibronektin kultiviert werden. Die zuletzt genannten drei Varianten eignen sich aber in jedem Fall nur für eine kurzfristige Kultur, da das Fehlen einer Kolllagenüberschichtung einen Zusammenbruch (bzw. Veränderung) des Zytoskeletts des Hepatozyten und damit der Morphologie dieser Zellen bedeutet. Durch die enge Verbindung von Morphologie und Funktion von Hepatozyten in Kultur ist ein derartiges Kultursystem von nur geringem kurzfristigem und keinerlei längerfristigem Nutzen.

Ein weiteres Problem der extrazellulären Matrix für bipolar adhärierte Hepatozyten 1 ist die geringe Stabilität gegenüber Scherkräften. Selbst unter statischen Kulturbedingungen kann sich insbensonders die bedeckende, obere Kollagenschicht ablösen da sie einer wässigren Phase (Kulturmedium) zugewandt ist. Werden die Zellen auf mikroporösen Membranen kultiviert, die gegebenenfalls die untere Matrixschicht 2 ersetzen kann und damit ein Teil des Sandwiches bildet, so kann ein Ablösen der Hepatozyten 1 auch von der Unterseite her erfolgen. Dies kann sowohl in Fällen auftreten in denen die Zellen direkt an der mikroporösen Struktur adhärieren oder wenn diese Membran mit einer Matrix vorbeschichtet wird.

Die Kultivierung der Hepatozyten kann entweder in einer Standardkultur in einem Kunststoff- oder Glasschälchen erfolgen, wobei die Sauerstoffzufuhr über das Kulturmedium 3 erfolgt oder in größerem Maßstab nach Art eines Bioreaktors, wobei schichtweise die Matrix 2 im Sandwich-verfahren jeweils auf einer gasdurchlässigen Membrane angeordnet ist. Ein derartiger "Bioreaktor" ist in der DE 42 06 585 beschrieben.

Der vorliegenden Erfindung liegt daher die Aufgabe zugrunde, ein rationelles Verfahren zu schaffen, bei dem der für Hepatozyten in der Sandwichtechnik bzw. Gel Entrapment Technik typische Funktionserhalt, trotz völligen Verzichtes auf die Zugabe von Serum oder anderen Zusätzen zu Standardkulturmedien möglich bleibt. Darüber hinaus soll ein mechanisch und biochemisch stabiles Kulturmodell mit höherer Reproduzierbarkeit der Ergebnisse erstellt werden.

Erfindungsgemäß wird diese Aufgabe durch die im kennzeichnenden Teil von Anspruch 1 genannten Merkmale gelöst.

Das erfindungsgemäße Vorgehen stellt somit eine Vereinfachung durch Limitierung auf die wesentlichen Komponenten dar.

Erfindungsgemäß kann eine Kombination von Fibronektin oder anderen Matrixkomponenten wie Kollagene (z.B. Typ III oder TypIV), Laminine, Vitronectin, Glycoproteine, Proteoglycane, Heparansulfat, Lipide, Linolensäure oder Lipopolysacharide zu der für die Sandwichtechnik bzw. Gel Entrapment Technik verwendeten Grundmatrix oder einem als Katalysator verwendeten Puffer (Mediumkonzentrat) durchgeführt werden.

Die extrazellulären Matrixkomponenten erzeugen einen besseren "in vivo-Effekt", denn sie haben induktive Faktoren für die Hepatozyten (Leberzellen).

Praktisch geht man von einer einfachen Grundmatrix aus und fügt dann bausteinartig einen Stoff nach dem anderen hinzu. In vielen Fällen wird dabei die Zugabe von Fibronectin ausreichend sein.

Die Grundmatrix, die hauptsächlich bzw. im wesentlichen aus Kollagen Typ 1 besteht, kann aus Rattenschwanzkollagen, Haut oder Knorbelgewebe präpariert werden.

Das erfindungsgemäße Verfahren hat weitreichende Vorteile, so wurde in eigenen Vorarbeiten Fibronektin als einer noch fehlenden Faktoren identifiziert, der bei der Sandwichtechnik oder Gel Entrapment Technik die völlig serumfreie Kultur insbesonders auch hinsichtlich eines langfristigen Funktionserhalts ermöglicht. Wird Fibronectin vorzugsweise direkt der für die Sandwichtechnik oder Gel Entrapment Technik verwendeten Matrix zugemischt, so befinden sich diese Moleküle direkt innerhalb des Adhäsionssubrats auf beiden Seiten der Zellen d.h. der Hepatozyten in Kontakt mit den sinusoidalen Seiten der Zellen. Durch den bei der Gelierung einsetzenden Vernetzungsprozess der Kollagenfibrillen werden die Fibronektinmoleküle innerhalb der Kollagenfibrillen zurückgehalten. Wird Fibronektin nur dem Überstand zugesetzt, so kann es indirekt in den stationären Matrixbereich diffundieren, wird jedoch zwangsweise bei den routinemäßig anfallenden Medienwechseln zu einem wesentlichen Teil wieder entfernt. Dennoch ist auch diese Methode schon bei einmaliger Gabe und entsprechend höherer Konzentration von Fibronektin ausreichend, um eine stabile, serumfreie Kultur von Hepatozyten bei Verwendung der Technik der bipolaren Adhäsion an extrazellulärer Matrix zu ermöglichen. Die Sandwichtechnik ist hierfür besser geeignet als die Gel Entrapment Technik, da das fibronektinhaltige Suspensionsmedium mit den Zellen aufgegeben werden kann, bevor die zweite Matrixschicht aufgetragen wird. Die Verwendung konventioneller Kulturmedien wie z.B. Williams E oder DMEM wird somit auch im Rahmen serumfreier Kulturen ermöglicht.

Eines der Vorteile der erfindungsgemäßen Form des Verfahren besteht somit in der stabilen Ausprägung der Grundlagen der längerfristigen serumfreien Kultur.

Dies führt zu einer Einsparung von Kosten, da das an sich teure Fibronektin innerhalb der bipolaren Matrix gebunden ist und nur einmal eingesetzt wird. Zweitens können in einen weiteren Vorteil der erfindungsgemäßen Form des Verfahrens nachfolgende Arbeiten mit beliebigen Medien durchgeführt werden. Für verschiedene Untersuchungen wurden spezielle Medien entwickelt, die hinsichtlich des zu erreichenden Ziels speziell adaptiert waren. Als Beispiel sei genannt, daß glucosefreie Medien zur Untersuchung der Gluconeogenese von Hepatozyten verwendet wurden. Sollen diese Untersuchungen nun auch in serumfreien Systemen durchgeführt werden, so müßte auf hierfür speziell definierte Medien wieder verzichtet werden und auf die sogenannten serumfreien Medien zurückgegriffen werden. Diese sind jedoch verständlicherweise nicht für derartige Spezialuntersuchungen konzipiert, sondern möglichst umfassend aufgebaut worden. Durch Ermöglichung der serumfreien Kultur allein durch die erfindungsgemäße Zusammensetzung des Adhäsionsubstrats im Rahmen der Sandwichtechnik oder Gel Entrapment Technik erhält der Untersucher eine bessere Flexibilität für seine Untersuchungen bei wesentlich überschaubareren und vereinfachten Kulturbedingungen. Die adhäsionsfördernde Wirkung von Fibronektin ist an sich bekannt. Die konventionelle Verwendung von Fibronektin ist in Zusammenhang mit Hepatozyten jedoch von keinem oder bestenfalls nur von kurzfristigen Vorteil.

Hepatozyten verlieren auf Einzelgel Unterlagen unabhängig von der Zusammensetzung des Kulturmediums (mit und ohne Serum) oder der Matrix (mit oder ohne Fibronektin) innerhalb weniger Tage ihre normale Morphologie und Funktionsfähigkeit. Die Verwendung des Begriffes serumfrei bedeutet in diesem Falle eher das Fehlen von FCS als die Ermöglichung von Grundlagen für die längerfristige serumfreie Kultur.

Die folgende Tabelle 1 faßt die Ergebnisse der Verwendung von FCS (fetal calf serum) und Fibronektin bei der Hepatozytenkultur hinsichtlich des Einflusses auf die Kulturdauer zusammen. Das an sich bekannte Sandwichkultursystem eignet sich bei der Verwendung von FCS Zusatz, der zumindest auf die Adhäsionsphase begrenzt sein muß, sowohl für kurzfristige (einige Tage) als auch eine längerfristige Kultur (Wochen).

Nur die Verbindung von Fibronektin und einer komplett serumfreien Sandwichtechnik führt zu einem auch längerfristig stabilen und reproduzierbaren Kultursystem. Ein derartiges Modell ist der bisher bekannten Sandwichtechnik überlegen, da ein wesentlich verläßlicheres Kulturmodell erstellt werden kann.

Die durch die Veränderung der Matrixzusammensetzung unter Zusatz definierter Komponenten erzielbare Verbesserung ist jedoch nicht nur auf eine höhere Reproduzierbarkeit beschränkt, wie z.B. das Studium von Wachstumshormonen. Durch die Kombination und Ermöglichung eines derartigen Ansatzes mit der Sandwichtechnik entsteht ein allen anderen Ansätzen (einschließlich der bisherigen Sandwich-technik) überlegenes Modell. Die Expression von Cytochrom P450, einem sehr empfindlichen Parameter der Kulturqualität, fällt in konventionellen Ansätzen zur Sandwichtechnik trotz der Stabilisierung der Albuminsekretion besonders initial ab. Bei einer Veränderung der Matrixzusammensetzung in Sinne der Erfindung durch Zusatz von Fibronektin, resultiert eine Stabilisierung der P450 Expression auch im Vergleich mit frischen Lebergewebe auf einem Level von 100 % selbst in längerfristigen Untersuchungen.

**Tabelle 1**

| | Kurzzeitfähigkeit | Langzeitfähigkeit |
|---|---|---|
| Sandwich + FCS | (+ +) | (+ +) |
| Sandwich - FCS | (+ +) | - |
| Sandwich + FN | ++ | ++ |
| Einzelgel + FCS | (+) | - |
| Einzelgel - FCS | + | - |
| Einzelgel + FN | + | - |
| () wegen mangelnder Reproduzierbarkeit von z.B. Untersuchung von Rezeptorvermittelten Prozessen wie z.B. Wachstumsfaktoren. | | |

Die Erfindung besteht also in der Verknüpfung mehrerer zum Teil bekannter Vorgehensweisen oder Substanzen (Sandwichtechnik/Gel Entrapment Technik/Fibronektin) und in einer speziellen Adaptation dieser Techniken für die primäre Hepatozytenkultur zu einem sinnvollen und bisher nicht bekannten Ganzen.

Erfindungsgemäß kann Fibronektin zu den nach der Sandwichtechnik/Gel Entrapment Technik kultivierten Hepatozyten auch zum Überstand hinzugegeben werden. Hierfür ist eine Einwirkzeit von einigen Stunden (nicht mehr als ca. 4 Std.) ausreichend, um auch dadurch eine stabile serumfreie Kultur mit ansonsten konventionellen Kulturmedien erreichen zu können (z.B. Williams E). Im Unterschied zur Zugabe von Fibronektin zur stationären Phase wie oben beschrieben ist dieses Verfahren für den Anwender/Verbraucher jedoch aufwendiger, da ein Medium mit Fibronektin für die Adhäsionsphase vorbereitet werden muß. Der ansonsten aus 2 Komponenten bestehende Kulturkit (Kollagen, Katalysatorkonzentrat, wobei eines von beiden die Matrixzusätze enthält) wird dadurch um eine dritte Komponente erweitert.

Im Unterschied zu bisherigen Verfahren die eine Veränderung/Induktion hepatozellulärer Funktionen allein durch den Informationsgehalt dieser Kofaktoren in Einzelgelsystemen zu erreichen versuchten, bildet die Basis des erfindungsgemäßen Verfahrens die Erkenntnis, daß die Position dieser Kofaktoren relativ zu den Hepatozyten einen weiteren wesentlichen Informationsgehalt für die Hepatozyten beinhaltet. Durch die Verwendung einer definierten und einfachen Grundmatix (z.B. Kollagen Typ I) für die Sandwichtechnik/Gel Entrapment Technik lassen sich somit stationäre Doppematrixsysteme unterschiedlicher Komplexizät systematisch aufbauen.

Erfindungsgemäß werden zusätzlich die für die Sandwichtechnik notwendigen Materalien in einem gebrauchsfertigen Kit zusammengesetzt. Dies bezieht sich auf die Anwendung dieser Erkenntnisse zur Herstellung eines entsprechend konfigurierten Hepatozytenkulturkits. Dieser besteht aus einer Kollagenlösung, einem Mediumkonzentrat eines Standardkulturmediums, die separat in zwei sterilen Behältern bei ca. 4 °C aufbewahrt werden können. Fibronektin kann entweder der Matrix oder dem Katalysatorpuffer zugesetzt werden. Alle Reagenzien können auch in Pulverform vorbereitet werden.

Erfindungsgemäß kann auch eine serumfreie Kokultur zwischen bipolar adhärierten Hepatozyten mit Zellen, die oberhalb der, die Hepatozyten bedeckenden, Matrixschicht kultiviert werden, vorgenommen werden. Dies dient der Durchführung des Studiums von Interaktionen in serumfreien Medien zwischen diesen Zellarten. Hierzu zählen toxikologische Untersuchungen und Mutagenitätsteste.

Ein weiterer Vorteil der Erfindung liegt in der Verwendbarkeit von Hepatozyten verschiedener Spezies wie auch z.B. humaner Hepatozyten, da die Matrixzusammensetzung in Abhängigkeit von der verwendeten Spezies adaptiert werden kann, um eine bessere Kulturstabilität zu erreichen.

Nachfolgend sind 4 Ausführungsbeispiele des erfindungsgemäßen Verfahrens prinzipmäßig erläutert.

Es zeigt:
- Fig. 1: eine bevorzugte Form bei direkter Zugabe von Matrixkomponenten zu einer Grundmatrix oder einem Katalysatorgemisch und Kombination mit der Sandwichtechnik;
- Fig. 2a: eine Situation während der Vorbereitung der Sandwichtechnik und Zugabe von Fibronektinmolekülen in das Medium;
- Fig. 2b: die Sandwichtechnik nach Fig. 2a mit einer zweiten, oberen Matrixschicht als stationäre Phase;
- Fig. 3: eine Form bei direkter Zugabe der Matrixkomponenten zur Grundmatrix oder dem Katalysatorgemisch und Kombination der Gel Entrapment Technik;
- Fig. 4: eine Situation bei Verwendung der Gel Entrapment Technik und Zugabe von Fibronektinmolekülen (F) in das Medium nach erfolgter Verfestigung der Grundmatrix.

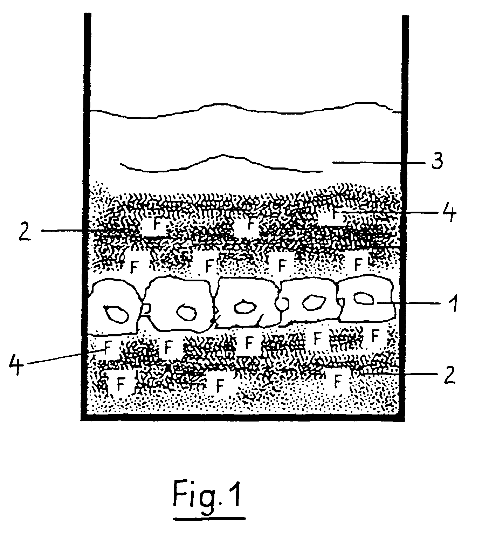

Gemäß Fig. 1 befinden sich Hepatozyten 1 innerhalb einer stationären Phase mit bipolarer (zweiseitiger, oben und unten) Adhäsion an einer extrazellulären Matrix 2. Einer Grundmatrix zugesetzte Matrixmoleküle F sind innerhalb dieser Matrix gebunden. Als Matrixmoleküle können z.B. Fibronektinmoleküle 4 vorgesehen sein. Dies bedingt eine Anreicherung der Fibronektinmoleküle innerhalb der die Hepatozyten 1 umgebenden fibrillären Matrix 2. Über der gebundenen Matrixkomponente, z.B. Fibronektinmoleküle 4, die in die stationäre Phase der Matrix 2 eingebettet ist, befindet sich ein Kulturmedium 3.

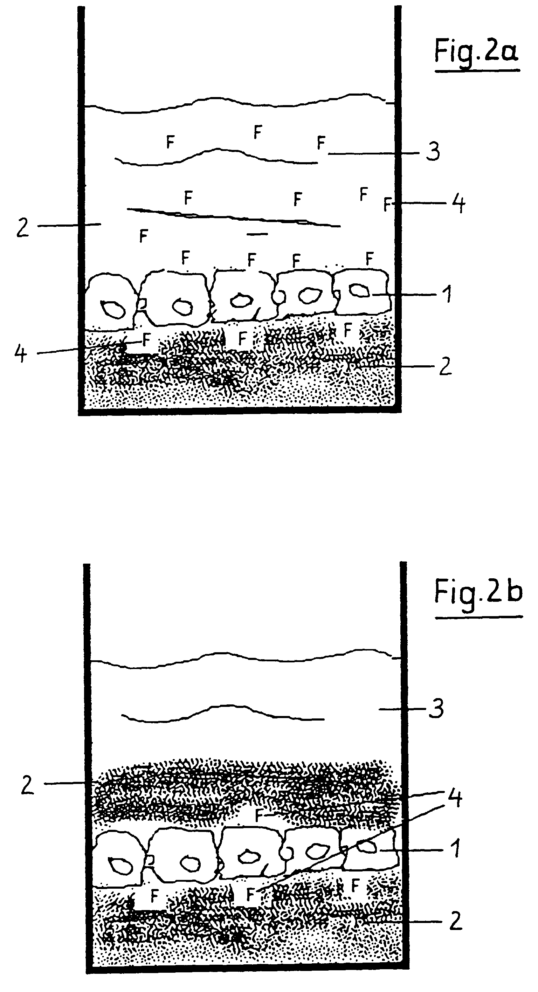

Gemäß Fig. 2a können Fibronektimoleküle 4 zumindest während der Adhäsionsphase direkt auf die Hepatozyten 1 einwirken, werden danach aber zum Zeitpunkt der Überschichtung mit der zweiten Matrixschicht entfernt, soweit sie nicht in die Matrix 2 diffundieren (siehe Fig. 2b). Oberhalb des Sandwiches befindet sich das Kulturmedium 3.

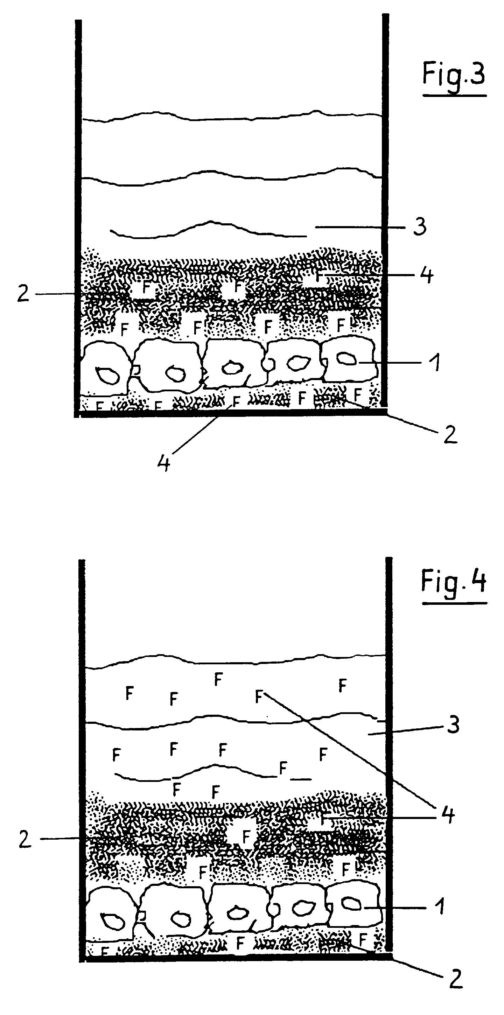

Wie in Fig. 3 dargestellt, befinden sich Hepatozyten 1 innerhalb der stationären Phase 2 mit bipolarer (zweiseitiger, oben und unten) Adhäsion an die extrazelluläre Matrix 2. Der Grundmatrix zugesetzte Matrixmoleküle (F) sind innerhalb dieser Matrix gebunden. Dies bedingt eine Anreicherung z.B. von Fibronektinmolekülen 4 innerhalb der die Hepatozyten 1 umgebenden fibrillären Matrix 2.

Wie aus Fig. 4 ersichtlich ist, erreichen Fibronektinmoleküle 4 indirekt die Hepatozyten 1, werden danach aber zum Zeitpunkt nachfolgender Medienwechsel wieder entfernt, soweit sie nicht in die Matrix 2 diffundieren. Fibronektinmoleküle 4 befinden sich auch in dem darüberliegenden Kulturmedium 3.

Anstelle oder zusätzlich zu den in den Ausführungsbeispielen dargestellten Fibronektinmolekülen 4 können auch andere bzw. weitere extrazelluläre Matrixkomponenten der vorstehend beschriebenen Art in die Kollagen- bzw. Matrixschicht 2 eingebracht werden.

Erfindungsgemäß können zur Kollagenmatrix weitere mechanisch stabilisierende Faktoren wie Ca-alginate, Fibrinogen, k-carrageenan, Agar, Agarose, Harze, Gelatine und gelbildende Substanzen, wie z.B. Carbopol (Polyakrylsäure), eventuell auch photopolymerisierende Harze und Polyurethan hinzugegeben werden. Weiterhin ist die Hinzumischung von Klebstoffen auf der Basis von Knochenleim, Hasenleim oder anderen biologischen aber auch nicht biologischen Ursprungsmateralien möglich, sofern diese nicht toxisch sind.

Wichtig hierbei ist nur, daß eine Polymerisation bzw. Verfestigung der bedeckenden Matrixschicht erst nach Ausbildung eines Hepatozytenmonolayers erfolgt (Sandwichtechnik/Gel Entrapment Technik). Dadurch sollen bei der Gel Entrapment Technik die Hepatozyten als Monolayer innerhalb von 2 Schichten eingebettet werden. Die Vorrichtung ist somit durch eine strikte Trennung der Hepatozyten- und Matrixschichtung gekennzeichnet entsprechend dem Prinzip der Sandwichtechnik und der modifizierten Gel Entrapment Technik bei der die Hepatozyten innerhalb der Matrix in einem Arbeitsgang aufgegeben werden und dann innerhalb der Matrix eine Sedimentation der Hepatozyten als Monolayer erfolgen kann, bevor eine Polymerisation der Matrix eintritt. Dies ist eine wichtiges Unterscheidungsmerkmal im Unterschied zu anderen Verfahren der Zellinkapsulation, die immer nur Zellen als Aggregate, Spheroide oder als isolierte Zellen inkapsulierten.

In einer weiteren vorteilhaften Form der Erfindung kann die zur Adähsion benötigte Matrix auch stabil, z.B. durch kovalente Bindungen oder durch vorhergehende Ionisation der Zellkulturmateralien (Membranen wie Polypropylen, Polyester, oder anderweitige Unterlagen wie z.B. Polysteren, Vicryl., Polykarbonate oder sonstige Kunststoffe, Glas) gebunden werden. Hierbei wäre eine Mischung aus Kollagen und Fibronektin besonders vorteilhaft. Es können so sehr stabile (Monate bis Jahre) Verbindung zwischen anorganischen Trägerflächen und organischen Molekülen zur Vorbereitung einer Sandwichtechnik/Gel Entrapment Technik bereitgestellt werden. Zur überschichtung kann dann wieder ein Kollagen-Fibronektin - gemisch mit und ohne Ca-Alginaten oder auch nur z.B. Ca-Alginate aufgegeben werden.

Im Gegensatz zu den bekannten und für Hepatozyten inetfizienten Techniken zur Verbesserung der Matrixbedingungen und Kulturbedingungen liegt erfindungsgemäß ein Verfahren vor, das durch Kombination der Technik der bipolaren Adhäsion von Hepatozyten an eine einfache Grundmatrix und der Zumischung von weiteren definierten Faktoren wesentliche Vorteile für die längerfristige Kultur dieser Zellen ermöglicht. Hierzu zählt z.B. die Ermöglichung einer völlig serumfreien Kultur von Leberzellen bei erhöhter mechanischer und biochemischer Stabilität des Kultursystems.

Es liegt nunmehr erfindungsgemäß ein einfach anzuwendendes Verfahren vor, das bei vollem Funktionserhalt in vitro die Verwendung von Standard-Kulturmedien für die serumfreie Kultur dieser Zellen ermöglicht und zur Herstellung eines entsprechend konfigurierten Zellkulturkits zur serumfreien Kultur mit konventionellen Medien geeignet ist.

## Patentansprüche

1. Verfahren zum Züchten von bipolar adhärierter Hepatozyten (1), insbesondere im Sachwichverfahren, wobei die Hepatozyten (1) in Kontakt der beiden sinusoidalen Seiten mit polarisiert zu den Hepatozyten angebrachter Matrix (2) liegen,
**gekennzeichnet** durch
die Verwendung von definierten Zusätzen extrazellulärer Matrixkomponenten (F) zu einer Grundmatrix, wodurch eine polarisierte Anreicherung und Bindung dieser Faktoren im stationären Matrixkontaktbereich der Hepatozyten (1) erreicht wird.

2. Verfahren nach Anspruch 1,
**dadurch gekennzeichnet**, daß
als Grundmatrix, hauptsächlich Kollagen, z.B. Typ I, verwendet wird.

3. Verfahren nach Anspruch 1 oder 2,
**dadurch gekennzeichnet,** daß
als extrazelluläre Matrixkomponenten (F), Fibronektin (4), Kollagene (z.B. Typ III oder Typ IV), Laminine, Vitronectin, Glycoproteine, Proteoglycane, Heparansulfat, Lipide, Linolensäure, Lipopolysacharide oder Mischungen daraus verwendet werden.

4. Verfahren nach einem der Ansprüche 1 bis 3,
**dadurch gekennzeichnet**, daß
mechanisch stabilisierende Komponenten verwendet werden.

5. Verfahren nach Anspruch 4,
**dadurch gekennzeichnet**, daß
als mechanisch stabilisierende Komponenten Ca-Alginate, Fibrinogen, k-carrageenan, Agar, Agarose, Harze, Polyurethan, Gelatine und gelbildende Substanzen, wie z.B. Carbopol (Polyakrylsäure), Knochenleim oder Hasenleim verwendet werden.

6. Verfahren nach einem der Ansprüche 1 bis 5,
**gekennzeichnet** durch
die Verwendung extrazellulärer Matrixkomponenten als definierter indirekter Zusatz zur Matrix bei Zugabe mit dem Kulturmedium (3) während der Adhäsionsphase und gleichzeitiger oder nachfolgender Kultivierung der Hepatozyten in Kontakt der beiden sinusoidalen Seiten mit extrazellulärer Matrix (2), ermöglicht entweder durch die sogenannte Sandwichtechnik oder alternativ durch die Gel Entrapment Technik.

7. Verfahren nach Anspruch 1,
**dadurch gekennzeichnet**, daß
extrazelluläre Matrixkomponenten (F) und/oder mechanisch stabilisierende Komponenten einem Katalysatorgemisch zugemischt werden, das aus einem Konzentrat, z.B. einem Nährmediumkonzentrat oder einer Pufferlösung, besteht.

8. Verfahren nach einem der Ansprüche 1 bis 7,
**dadurch gekennzeichnet**, daß
die für die Kultur bipolar adhärierter Hepatozyten (1) notwendigen Reagenzien und Protokolle bei Verwendung der durch diese Zusätze verbesserten Grundmatrix (2) in einem Kit zusammengestellt werden, bestehend aus einer Grundmatrix (2) und einem Katalysatorgemisch, wobei entweder eines oder beide dieser Substanzen extrazellulärer Matrixkompnenten nach Anspruch 3 und/oder stabilisierende Komponenten nach Anspruch 4 oder 5 enthalten.

9. Verfahren nach einem der Ansprüche 1 bis 8,
**dadurch gekennzeichnet**, daß
die für die Kultur bipolar adhärierter Hepatozyten (1) notwendigen Reagenzien und Protokolle bei Verwendung der durch die extrazellulären Matrixkomponenten verbesserbaren Grundmatrix (2) in einem Kit zusammengestellt werden, bestehend aus einer Grundmatrix (2) und einem Katalysatorgemisch, wobei keines dieser Substanzen die Zusätze enthalten und diese dann separat als drei Komponente des Kits angeboten werden.

10. Verfahren nach Anspruch 8 oder 9,
**dadurch gekennzeichnet**, daß
die für den Kit zusammenzustellenden Basisreagenzien aus Rattenschwanzkollagen, Knorpelgewebe oder Hautkollagen hergestellt werden und daß das Katalysatorgemisch ein 10 fach Konzentrat eines beliebigen oder sonst nicht für die serumfreie Kultur von Hepatozyten geeigneten Mediums darstellt.

11. Verfahren nach Anspruch 1,
**dadurch gekennzeichnet**, daß
die serumfreie Kokultur von bipolar adhärierten Hepatozyten (1) entweder entsprechend der Sandwichtechnik oder der Gel Entrapment Technik und einer weiteren Zellart mit Zellkulturen unter Zusatz von Fibronektin (4) durchgeführt wird.

## Claims

1. Method for the culture of bipolar-adhesion hepatocytes (1), in particular by the sandwich method, the two sinusoidal sides of the hepatocytes (1) being in contact with a matrix (2) added in a polarised manner relative to the hepatocytes, characterised by the use of defined additions of extracellular matrix components (F) to a basic matrix, thereby resulting in the polarised enrichment and bonding of these factors in the stationary matrix contact region of the hepatocytes (1).

2. Method according to claim 1, characterised in that principally collagen, e.g. type I collagen, is used as the basic matrix.

3. Method according to claim 1 or claim 2, characterised in that fibronectin (4), collagens (e.g. type III or type IV collagen), laminins, vitronectin, glycoproteins, proteoglycans, heparan sulphate, lipids, linolenic acid, lipopolysaccharides or mixtures thereof are used as the extracellular matrix components (F).

4. Method according to one of claims 1 to 3, characterised in that mechanically stabilising components are used.

5. Method according to claim 4, characterised in that Ca alginates, fibrinogen, k-carrageenan, agar, agarose, resins, polyurethane, gelatin and gel-forming substances, e.g. carbopol (polyacrylic acid), bone glue or hare glue are used as the mechanically stabilising components.

6. Method according to one of claims 1 to 5, characterised by the use of extracellular matrix components as a defined indirect addition to the matrix when added with the culture medium (3) during the adhesion phase and the simultaneous or subsequent cultivation of the hepatocytes with the two sinusoidal sides in contact with the extracellular matrix (2), made possible either by what is referred to as the sandwich technique or alternatively by the gel entrapment technique.

7. Method according to claim 1, characterised in that extracellular matrix components (F) and/or mechanically stabilising components are added to a catalyst mixture consisting of a concentrate, e.g. a culture medium concentrate or a buffer solution.

8. Method according to one of claims 1 to 7, characterised in that the reagents required for the culture of bipolar-adhesion hepatocytes (1) and the protocols when using the basic matrix (2) improved by these additives are assembled in a kit consisting of a basic matrix (2) and a catalyst mixture, either one or both of these substances containing extracellular matrix components according to claim 3 and/or stabilising components according to claim 4 or claim 5.

9. Method according to one of claims 1 to 8, characterised in that the reagents required for the culture of bipolar-adhesion hepatocytes (1) and the protocols when using the basic matrix (2) improved by the extracellular matrix components are assembled in a kit consisting of a basic matrix (2) and a catalyst mixture, none of these substances containing the additives and these then being provided separately as three components of the kit.

10. Method according to claim 8 or claim 9, characterised in that the basic reagents to be assembled for the kit are produced from rat's tail collagen, cartilaginous tissue or skin collagen and that the catalyst mixture is a tenfold concentrate of any desired medium or a medium otherwise not suitable for the serum-free culture of hepatocytes.

11. Method according to claim 1, characterised in that the serum-free co-culture of bipolar-adhesion hepatocytes (1) is carried out either according to the sandwich technique or the gel entrapment technique and another type of cell with cell cultures by adding fibronectin (4).

## Revendications

1. Procédé de culture d'hépatocytes à adhérence bipolaire (1), en particulier dans un procédé sandwich, dans lequel les hépatocytes (1) sont en contact des deux côtés sinusoïdaux avec une matrice (2) déposée de manière polarisée sur les hépatocytes,
**caractérisé** par,
l'utilisation d'adjonctions définies de composants de matrice extracellulaire (F) à une matrice de base de manière à obtenir une concentration polarisée et une liaison de ces facteurs dans la zone de contact de la matrice stationnaire des hépatocytes (1).

2. Procédé selon la revendication 1,
**caractérisé en ce que** l'on utilise comme matrice de base essentiellement du collagène, par exemple du type I.

3. Procédé selon la revendication 1 ou 2,
**caractérisé en ce que** l'on utilise comme composants de matrice extracellulaire (F) de la fibronectine (4), du collagène (par exemple du type III ou du type IV), de la laminine, de la vitronectine, des glycoprotéines, du protéoglycane, de l'héparane sulfate, des lipides, de l'acide linolénique, des lipopolysaccharides ou des mélanges de ceux-ci.

4. Procédé selon l'une quelconque des revendications 1 à 3,
**caractérisé en ce que** l'on utilise des composants stabilisateurs mécaniques.

5. Procédé selon la revendication 4,
**caractérisé en ce que** l'on utilise comme composants stabilisateurs mécaniques de l'alginate de Ca, du fibrinogène, du carragénane-k, de l'agar, de l'agarose, des résines, du polyuréthane, de la gélatine et des substances formant un gel, telles que le carbopol (acide polyacrylique), de l'ostéocolle ou de la colle de lapin.

6. Procédé selon l'une quelconque des revendications 1 à 5,
**caractérisé** par
l'utilisation de composants de matrice extracellulaire comme addition définie indirecte à la matrice par ajout avec le milieu de culture (3) pendant la phase d'adhésion et simultanément ou consécutivement à la culture des hépatocytes en contact avec les deux côtés sinusoïdaux avec la matrice extracellulaire (2), rendue possible soit par la technique dite sandwich ou autrement par la technique de piégeage par gel.

7. Procédé selon la revendication 1,
**caractérisé en ce que** les composants de matrice extracellulaire (F) et/ou les composants stabilisateurs mécaniques sont mélangés à un mélange catalyseur, qui se compose d'un concentré, par exemple un concentré de milieu nutritif ou une solution tampon.

8. Procédé selon l'une des revendications 1 à 7,
**caractérisé en ce que** les réactifs nécessaires à la culture des hépatocytes à adhérence bipolaire (1) et les protocoles sur l'utilisation de la matrice de base améliorée par ces additions sont réunis dans un kit, composé d'une matrice de base (2) et d'un mélange catalyseur, soit l'une soit les deux substances renfermant des composants de matrice extracellulaire selon la revendication 3 et/ou des composants stabilisateurs selon la revendication 4 ou 5.

9. Procédé selon l'une des revendications 1 à 8,
**caractérisé en ce que** les réactifs nécessaires à la culture des hépatocytes à adhérence bipolaire (1) et les protocoles pour l'utilisation de la matrice de base pouvant être améliorés grâce aux composants de matrice extracellulaire sont réunis dans un kit, composé d'une matrice de base (2) et d'un mélange catalyseur, aucune de ces substances ne contenant les additifs, ceux-ci étant proposés séparément sous forme de trois composants du kit.

10. Procédé selon la revendication 8 ou 9,
**caractérisé en ce que** les réactifs de base à réunir pour le kit sont fabriqués à partir de collagène de queue de rat, de tissu cartilagineux ou de collagène de la peau, et en ce que le mélange catalyseur représente un concentré 10 fois d'un milieu quelconque d'un milieu non approprié à la culture sans sérum des hépatocytes.

11. Procédé selon la revendication 1,
**caractérisé en ce que** la co-culture sans sérum des hépatocytes à adhérence bipolaire (1) est effectuée soit selon la technique sandwich, soit selon la technique de piégeage par gel et un autre type de cellule avec des cultures de cellules par adjonction de fibronectine (4).
